# EUROPEAN PATENT SPECIFICATION

(11) **EP 1 178 834 B1**
(45) Date of publication and mention of the grant of the patent: **04.01.2006**
(21) Application number: 99915970.0
(22) Date of filing: 22.04.1999
(51) Int. Cl.: A61K 47/48, A61K 38/39, A61K 38/18, A61K 38/36, A61P 17/00

(54) **CONTROLLED RELEASE OF GROWTH FACTORS FROM HEPARIN CONTAINING MATRICES**
KONTROLLIERTE ABGABE DER WACHSTUMSFAKTOREN VON HEPARIN ENTHALTENDEN MATRIZEN
DIFFUSION REGULEE DE FACTEURS DE CROISSANCE DE MATRICES CONTENANT DE L'HEPARINE

(43) Date of publication of application: 13.02.2002
(73) Proprietor: Eidgenössische Technische Hochschule (ETH), 8092 Zürich (CH); Universität Zürich, 8006 Zürich (CH)
(72) Inventor: SAKIYAMA-ELBERT, Shelly, E., St. Louis, MO 63130 (US); HUBBELL, Jeffrey, A., CH-8126 Zurich (CH)
(74) Representative: Klunker . Schmitt-Nilson . Hirsch
(86) International application number: PCT/IB1999/000800
(87) International publication number: WO 2000/064481

(56) References cited:
- EP-A- 0 732 105
- EP-A- 0 838 219
- WO-A-00/13710
- WO-A-92/09301
- WO-A-96/16983
- WO-A-99/21588
- WO-A-99/54359
- DE-A- 19 841 698
- SAKIYAMA S. E. ET AL: "Incorporation of heparin-binding peptides into fibrin gels enhances neurite extension: an example of designer matrices in tissue engineering" FASEB, vol. 13, December 1999 (1999-12), pages 2214-2224, XP002128749
- SCHROEDER-TEFFT J. A. ET AL: "Collagen and heparin matrices for growth factor delivery" JOURNAL OF CONTROLLED RELEASE, vol. 49, 1997, pages 291-298, XP000867263
- SCHENSE J. C. ET AL: "Cross linking exogenous bifunctional peptides into fibrin gels with factor XIIIa" BIOCONJUGATE CHEMISTRY, vol. 10, no. 1, 12 August 1998 (1998-08-12), pages 75-81, XP000867031
- NIMNI M E: "Polypeptide growth factors: targeted delivery systems" BIOMATERIALS,GB,ELSEVIER SCIENCE PUBLISHERS BV., BARKING, vol. 18, no. 18, page 1201-1225 XP004086390 ISSN: 0142-9612
- HUBELL J. A.: "Bioactive biomaterials" CURRENT OPINION IN BIOTECHNOLOGY, vol. 10, April 1999 (1999-04), pages 123-129, XP000866483
- FOWLKES J L ET AL: "Characterization of glycosaminoglycan-binding domains present in insulin-like growth factor-binding protein-3." JOURNAL OF BIOLOGICAL CHEMISTRY, (1996 JUN 21) 271 (25) 14676-9., XP000867125
- CAMPBELL P G ET AL: "Insulin-like growth factor-binding protein-3 binds fibrinogen and fibrin." JOURNAL OF BIOLOGICAL CHEMISTRY, (1999 OCT 15) 274 (42) 30215-21. , XP000913749
- AMERSHAM BIOSCIENCES: "Leaflet for HiTrap Heparin HP" 2002, pages 1-5,

## Description

### FIELD OF THE INVENTION

The present invention relates to the field of three-dimensional matrices that contain pharmacoloeically active molecules, particularly growth factors. The invention also relates to the use of growth factors or proteins in a matrix designed to promote cell and tissue growth. The invention further relates to the use of growth factors with low heparin-binding affinity. In addition, the invention relates to the field of articles of manufacture useful as implantable devices and wound dressings as the matrix of the invention is designed to be used in conjunction with such devices to provide protracted and controlled release of growth factor, thus promoting wound healing in the patient.

### BACKGROUND

Many growth factors are thought of as "heparin-binding" growth factors. Families with one or more members that bind heparin include fibroblast growth factors and bone morphogenetic proteins (BMPs) (1, 2). Additional growth factors that bind heparin include transforming growth factor β1 (TGF-β1), interleukin-8, neurotrophin-6, vascular endothelial cell growth factor, heparin-binding epidermal growth factor, hepatocyte growth factor, connective tissue growth factor, midkine, and heparin-binding growth associate molecule (3-11). These factors have shown the potential to enhance healing in many different types of tissue including vasculature, skin, nerve, and liver.

Controlled delivery devices based on heparin-affinity of these growth factors have been designed previously (12-14). These drug delivery devices have previously been used to deliver "heparin-binding" growth factors. Such "heparin-binding" growth factors are typically considered to be those which bind to heparin with a relatively high affinity, often characterized by elution from heparin-affinity columns at NaCl concentrations well above physiological levels (>140 mM). In such delivery systems, the heparin-binding affinity of the growth factor is usually used to sequester the growth factors to immobilized heparin of some form. For example, Edelman et al. have used heparin-conjugated Sepharose beads to bind basic fibroblast growth factor (bFGF) and then encapsulated the beads with alginate (12. 19). These beads serve as reservoirs that release bFGF slowly based on the binding and dissociation constants of bFGF and heparin.

The delivery of "non-heparin-binding growth factors" has previously required release methods for delivery typically based on diffusion-controlled release of the factors from porous materials (15-18). There remains a need in the medical arts for a device that is capable of providing the release of low heparin-binding growth factors at a controlled and predictable rate in order to provide effective release of the factor over a clinically useful period during the wound healing process.

WO-A-99/54359 (state of the art within the sense of Art. 54 (3) EPC) discloses a matrix for purifying proteins by affinity chromatography comprising a heparin-sepharose gel having a low-affinity heparin-binding protein (IGF-1) and a high-affinity heparin-binding protein (MBF-2) bound thereto. EP-A-0 838 219 and EP-A-0 732 105 disclose a controlled released formulation comprising a low-affinity heparin-binding growth factor and heparin or heparin-like otigosaccharide entrapped in a collagen matrix. M. E. Nimni (1997) Biomaterials 18, 1201-1225 discloses a similar controlled release formulation comprising a low-affinity heparin-binding growth factors entrapped in a hydrogel matrix. WO-A-92/09301 discloses a formulation for promoting wound healing comprising a heparin-binding growth factor, heparin and fibrin glue. In none of these matrices is heparin (or heparin-like polysaccharide or polymer) bound to a peptide, which in turn is covalently bound to the substrate and has a high-affinity heparin-binding domain.

### SUMMARY OF THE INVENTION

In a general and overall sense, the present invention relates to the use of how non-heparin-binding growth factors in delivery techniques employing growth factors with heparin-affinity by utilizing low heparin affinity sequences present in many proteins. The particular growth factors employed as part of the invention have been found by the present inventors to possess a basic sequence at a site in the protein that is freely accessible in the proteins native conformation. This basic region may possess only relatively low heparin-affinity.

As used in the description of the present invention, "low-heparin-binding affinity" of a growth factor or peptide fragment thereof is defined as any protein, peptide, or derivative or combination thereof, that is capable of demonstrating the biological activity of a growth factor, and that has a relatively binding low affinity for binding heparin, and will elute from a heparin-affinity column at sub-physiological NaCl concentrations. Physiological levels of NaCl may be defined as about 140 mM NaCl. Herein the term "sub-physiological" levels of NaCl, therefore, may be further defined as from between about 25 mM to about 140 mM NaCl. Although low heparin-binding affinity growth factors elute from heparin-affinity columns at sub-physiological NcCl concentrations, their low affinity for heparin can still be used to sequester the protein or peptide to a matrix that contains heparin or a heparin-binding site.

By way of example, and in no way intending to be limited to any particular mechanism of action, the invention may be described as employing a matrix having growth factor proteins with a relatively high ratio of heparin-binding sites. A ratio of at least 1:1 heparin to growth factor must be used, but the greater the excess of heparin sites the slower the release. In this fashion, primarily "non-heparin-binding" growth factors or peptide fragments thereof with relatively low heparin-binding affinity can be bound to a heparin-decorated matrix. These matrices can then serve as reservoirs containing the growth factor or factors to be delivered. The dissociation kinetics of low affinity heparin-binding proteins are relatively fast, but the high number of binding sites allows rebinding of the growth factor before it can diffuse out of the matrix. Release can occur by diffusion of the growth factor out of the matrix prior to rebinding, or it can occur if the growth factor encounters a cell surface receptor before rebinding to a heparin site. In this fashion, release of the growth factor or bioactive fragment thereof can be sustained, and continue to foster improved healing.

In a general and overall sense, the present invention describes in at least one aspect a specially designed matrix that provides for the release of growth factors or bioactive fragments thereof. The growth factor is defined as having low binding affinity for heparin. The matrix, more particularly, may be defined as comprising a substrate capable of providing attachment of heparin, a heparin-like polysaccharide, or a heparin-like polymer, and a growth factor or peptide fragment thereof having a basic domain that binds heparin with low affinity.

The characteristic of the growth factor or peptide fragment thereof as binding to heparin with low affinity may be further described as a peptide/protein that will elute from a heparin affinity column at an NaCl concentration of about 25 mM to about 140 mM.

The "low heparin-binding affinity" growth factor or peptide fragment thereof may be further defined as comprising a length of about 8 to 30 amino acid residues: This sequence of amino acid residues, in some embodiments, may be defined as comprising at least 2 basic amino acid residues, a ratio of basic to acidic amino acid residues of at least 2, and a ratio of hydrophobic amino acid residues to basic amino acid residue of at least 0.67. The growth factor or fragment thereof elutes from a heparin affinity column at less than 140 mM or at about 25to about140 mM NaCl.

For purposes of this application, basic amino acids may be defined as K (lysine) or R (arginine). The acidic amino acid residues may be further defined as D (aspartic acid) or E (glutamic acid). The hydrophobic amino acid residues may be defined as A (alanine), V (valine), F (phenylalanine), P (proline), M (methionine), I (isoleucine), or L (leucine). For purposes of this application, C (cysteine) that are involved in a disulfide bridge are also considered hydrophobic.

By way of example, the low heparin-binding affinity growth factor or a peptide fragment thereof as defined in the invention comprises neunurin, persephin, IGF-1A, IGF-1 p, EGF, NGFβ, NT-3, BDNF, NT-4, TGF-β2, TGF-β3, TGF-β4, or a peptide fragment of any one of these. Other growth factors may be found which contain similar basic domains that are not enumerated here. The matrix itself may also comprise any of a variety of materials, such as fibrin, collagen, hyaluronic acid, or a synthetic polymer hydrogel. By way of example, the synthetic polymer hydrogel may be a poly (ethylene glycol) hydrogel or a derivative, thereof. Other synthetic polymer hydrogels may be used apart from those enumerated here.

The peptides of the invention that bind heparin with high affinity have a characteristic amino acid domain that will not elute from a heparin-affinity column at less than 140 mM NaCI. While many potential peptides exist, the inventors have identified several peptide sequences in particular. These are exemplified in the amino acid sequences identified in SEQ. ID. NO.: I, SEQ ID. NO.: 2: SEQ ID. NO.: 3; SEQ ID. NO.: 4; and SEQ ID. NO.: 5. Many other peptides may be used apart from the specifically enumerated sequences here.

The heparin or heparin-like polysaccharides of the invention may be further characterized, at least in some embodiments, as having a molecular weight of at least 3,000 Daltons. It is contemplated that virtually any molecular weight heparin or heparin-like polysaccharide could be used in the practice of the invention of at least 3,000 Daltons, without any upper molecular weight limitation. For practical purposes a molecular weight maximum of 10.000,000 may be considered. In particular applications, the heparin-like polysaccharide may he further defined as a polysaccharide having a molecular weight of at least 3,000 Daltons and having at least one negative charge per two saccharide rings and no more than one positive charge per ten saccharide rings.

Examples of heparin-like polysaccharide include dextran sulfate, chondroitin sulfate, heparin sulfate, fucan, alginate or derivatives thereof. The present inventors have found that particular preparations of the matrix that include a particular molar ratio of heparin to growth factor, such as a molar ratio of 1, may be employed in the practice of the invention. It has further been found that a matrix of the invention that includes a molar ratio of covalently-attached peptide having a binding domain that binds heparin with high affinity to heparin or a heparin-like polysaccharide of at least one is in some embodiments of the matrix a preferred ratio. These heparin and heparin-like polysaccharides may be either covalently attached to the substrate or immobilized via non-covalent interactions (i.e. electrostatically bound). Synthetic polymers may be designed that function in a heparin-like manner.

The substrate of the matrix as defined in the present invention may comprise fibrin, collagen, hyaluronic acid, a synthetic polymer gel, a mixture thereof, or any variety of synthetic derivatives thereof, that is capable of supporting the attachment of the types of peptides described there, and/or cells.

The matrix and various embodiments of the matrix described herein provide a multitude of advantages, particularly when employed at tissue sites where a wound healing response is desired. The growth factor or peptide fragment thereof provided in the matrix is released by the degradation of a component of the matrix, by the disassociation of growth factor from the heparin or heparin-like polysaccharide, or by a combination of these mechanisms. In this manner, a more sustained and controlled, release of growth factor into the site where the matrix is implanted may be achieved. Methods for providing the controlled release of growth factor at a wound site in need thereof are provided by the present invention. The growth factor for such applications may include TGF-β3, which may be particularly useful in dermal healing. The invention provides various articles of manufacture, such as a vascular graft or shunt, or tissue replacement that includes the matrix defined in this disclosure. Such articles may in some embodiments be defined as implantable sterilized compositions. The heparin-binding peptide used in the present examples possesses high heparin affinity.

In addition to using protein matrices as the substrate of the delivery system, other types of matrices can be used. Synthetic polymer hydrogels, including hydrogels formed by photopolymerization or conjugate addition reactions, can be utilized as the substrate for the delivery device. This synthetic material may contain cell adhesion domains, substrates for enzymatic degradation or hydrolysis, heparin-binding domains, or covalently bound heparin. Through either the covalent or non-covalent attachment of heparin, such synthetic matrices can bind low heparin-binding affinity growth factor proteins and release them in a controlled manner. Release can occur by degradation of matrix components or dissociation of the low heparin-binding affinity growth factor proteins, just as in protein matrices.

The substrate for the delivery system can also include matrices of hyaluronic acid or hyaluronic acid derivatives. Such materials are commonly used and readily available. The addition of covalently or non-covalently bound heparin or heparin-like polysaccharides can be used to provide controlled delivery of low heparin-binding affinity growth factor proteins. Release can occur by degradation of matrix components or dissociation of the low heparin-binding affinity growth factor proteins, just as in protein or synthetic polymer matrices.

In addition to heparin, other heparin-like polymers have similar binding affinity for heparin-binding proteins and peptides. Such heparin-like polymers include, for example, dextran sulfate, chondroitin sulfate, heparin sulfate, fucan and alginate (See Maaroufi, et al, 1997 (46) and Logeart, et al., (1997) (47). In addition, synthetic heparin-like polymers or polysaccharide derivatives also exist, which have similar binding affinity for heparin-binding proteins or peptides as heparin. Examples of heparin-like polysaccharide derivatives include dextran derivatives such as those made by de Raucourt, et al., (1998) (48) and Bagheri-Yamand, et al., (1998) (49). Examples of heparin-like synthetic polymers include those by Silver, et al., (1992) (50). For the purposes of this invention, usage of the term "heparin" is considered to include all heparin-like polymers and polysaccharides including those described above.

### (1) Amino acid three-letter and one-letter abbreviations:

| Abbreviation 3 letter | Abbreviation 1 letter | Amino Acid Name |
|---|---|---|
| Ala | A | Alanine |
| Arg | R | Arginine |
| Asn | N | Asparagine |
| Asp | D | Aspartic Acid |
| Cys | C | Cysteine |
| Glu | E | Glutamic Acid |
| GIn | Q | Glutamine |
| Gly | G | Glycine |
| His | H | Histidine |
| Ile | I | Isoleucine |
| Leu | L | Leucine |
| Lys | K | Lysine |
| Met | M | Methionine |
| Phe | F | Phenylalanine |
| Pro | P | Proline |
| Ser | S | Serine |
| Thr | T | Threonine |
| Trp | W | Tryptophan |
| Tyr | Y | Tyrosine |
| Val | V | Valine |

The following sequences are referenced in the description of the present invention.

| | | |
|---|---|---|
| SEQ ID NO: | 1 ATIII domain | K(βA)FAKLAARLYRKA |
| SEQ ID NO: 2 | PF4 domain . | YKKIIKKL |
| SEQ ID NO: 3 | NCAM domain | KHKGRDVILKKDVR |
| SEQ ID NO: 4 | ATIII domain modified | R(βA)FARLAARLYRRA |
| SEQ ID NO: 5 | bFGF domain | KDPKRLYRSRKY |
| SEQ ID NO: 6 | NGF basic domain | CVLSRKAVRRA |
| SEQ ID NO: 7 | NT-3 basic domain | CALSRKIGRT |
| SEQ ID NO: 8 | BDNF basic domain | CTLTIKRGR |

### BRIEF DESCRIPTON OF THE DRAWINGS

### Fig. 1

Effect of matrix bound basic fibroblast growth factor on neurite extension from dorsal root ganglia in three-dimensional fibrin gels. Ganglia were dissected from day 8 chick embryos and placed in prewashed fibrin gels. * denotes p<0.05 versus unmodified fibrin. The soluble treatments contain bFGF, but not heparin or heparin-binding peptide. These treatments show no difference from unmodified fibrin suggesting that the washing protocol used was sufficient to remove unbound growth factor. The results also suggest that peptide, heparin, and growth factor is all necessary components of the controlled release system.

Bar 1, fibrin; Bar 2, with Peptide + heparin; Bar 3, 0.1, µg/ml bound; Bar 4, 1.0 µg/ml bound; Bar 5, 1.0 µg/ml soluble; Bar 6, 5.0 µg/ml bound; Bar 7, 5.0 µg/ml bound; Bar 8, 1.0 µg/ml culture; Bar 9, VEGF 1.0 µg/ml; Bar 10, 1.0 µg/ml bound to 50% peptide; Bar 11. 1 µg/ml + soluble ATIII; Bar 12, 1 µg/ml + heparin (no peptide). "Bound" refers to fibrin, with Peptide and heparin. "Soluble" refers to fibrin.

### Fig. 2

Effect of matrix bound NGF-β, NT-3 and BDNF on neurite extension from dorsal root ganglia in three-dimensional fibrin gels. Ganglia were dissected from day 8 chick embryos and placed in prewashed fibrin gels. * denotes p<0.05 versus unmodified fibrin. The soluble treatments contain growth factor, but not heparin or heparin-binding peptide. These treatments show no difference from unmodified fibrin suggesting that the washing protocol used was sufficient to remove unbound growth factor.
Bar 1, fibrin; Bar 2, NGF bound at 0.1µg/ml; Bar 3, NGF soluble at 0.1µg/ml; Bar 4 BDNF bound at 0.1µg/ml; Bar 5, BDNF soluble at 0.1, µg/ml bound; Bar 6. NT-3 bound at 1.0 µg/ml; Bar 7, NT-3 soluble at 0.1 µg/ml bound. "Bound" refers to fibrin, with Peptide and heparin. "Soluble" refers to fibrin.

### Fig. 3

Ability of matrix bound NGF-β to promote neurite extension from dorsal root ganglia in three-dimensional fibrin gels as a function of wash time prior to cell seeding. Ganglia were dissected from day 8 chick embryos and placed in prewashed fibrin gels. * denotes p<0.05 versus unmodified fibrin. The soluble treatments contain growth factor, but not heparin or heparin-binding peptide. This assay demonstrates that the matrix bound NGF-β maintains its activity for 4 days while the unbound NGF-β is not active after 1 day of washing.
--■-- fibrin; --◆-- ngf bound (100 ng/ml); --●-- ngf soluble (100 ng/ml)

### DETAILED DESCRIPTION OF THE PREFERRED EMBODIMENTS

The present invention provides compositions useful in promoting the controlled release of low affinity-heparin-binding growth factors having a low heparin-binding domain of about 8 to about 30 basic amino acids in length.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1 -HEPARIN-AFFINITY CHROMATOGRAPHY OF HEPARIN-BINDING AND LOW AFFINITY HEPARIN-BINDING GROWTH FACTORS

Heparin-affinity chromatography is a method commonly used to determine the relative affinity of heparin-binding proteins. If a protein elutes at NaCl concentrations near or below physiological level (approximately 140 mM) it is not to be considered "heparin-binding" for purposes of the description of the present invention. This is because the growth factors would dissociate rapidly from heparin *in vivo.*

The relative affinity for heparin of proteins was determined by heparin-affinity chromatography, using a TSK-GEL Heparin-5PW (7.5 cm x 7.5 mm ID) column (TosoHass, Stuttgart, Germany). Samples of the protein were injected in 20 mM Tris, pH 7.4. 0.05 M NaCl. Elution was accomplished by running a gradient of NaCl from 0.05 M to 2.0 M over 30 min, and the NaCl concentration at which elution was observed was taken as a measure of the heparin-binding affinity of the protein. The relative heparin-binding affinity of proteins not previously reported in the literature to be heparin-binding was determined and compared with proteins such as bFGF and antithrombin III, which are know to be strongly heparin-binding. The results are shown in Table 1.

The two "non-heparin-binding" growth factors, TGF-β2 and NGFβ both eluted at sub-physiological NaCl concentrations, suggesting that they have relatively low heparin-binding affinity, and will rapidly dissociate from heparin under physiological conditions. The two heparin-binding proteins, antithrombin III and bFGF, elute at NaCl concentrations that are much greater than physiological levels.

Others have also used heparin to increase the activity of and prevent the degradation of growth factors. Schroeder et al. (20) used heparin to increase the stability of TGF-β1 and prevent loss of activity. They have also attached heparin to collagen and employed the heparin-TGF-β1 complex in collagen gels to show that such heparin-based systems can improve growth factor activity in vivo by controlled release (21). TGF-β1 is known to possess strong heparin binding affinity. However, such heparin-based release systems have not been tested previously with growth factors, which are considered to have low heparin-binding affinity (those which are characterized by elution from heparin-affinity columns at sub-physiological NaCl concentrations).

Analysis of the primary sequence of proteins by the present inventors, including growth factors, has revealed that the primary sequence may contain regions that are basic in nature and as that the basic residues are commonly flanked by hydrophobic residues. These sequences are generally of a similar nature to the XBBXBX heparin-binding consensus (where X is a hydropathic residue and B is a basic residue) the heparin-binding consensus was described by Cardin and Weintraub (22). However, the exact sequence of the basic regions vary from protein to protein. The three-dimensional structure for many proteins is also available, which allows the location of basic regions to be determined. In order for basic regions to be useful for sequestration of a protein, they must be located on the surface of the protein. Frequently, such regions are observed to occur in the amino or carboxy terminus of the protein.

**Table 1: NaCl concentration required to elute proteins from a heparin-affinity column.**

| Protein | [NaCl] require to elute peptide |
|---|---|
| bFGF | 2.0 M |
| antithrombin III | 1.58 M |
| TGF-β2 | 0.05 M |
| NGFβ | 0.05 M |

### EXAMPLE 2 - IN VITRO MODEL FOR BIOACTIVITY - NEURITE EXTENSION

The present example is provided to describe the model for assaying *in vitro* bioactivity of materials described herein.

A three-dimensional fibrin gel *in vitro* model for assay neurite extension. Fibrinogen was dissolved in water and dialyzed into Tris-buffered saline (TBS) at pH 7.4 for 24 hr. The concentration of fibrinogen was determined by measuring the absorbance at 280 nm. Fibrin gels were made containing a final concentration of 3.5 mg/ml fibrinogen, 2.5 mM Ca⁺⁺, and 2 NIH units/ml thrombin in TBS. The polymerization mixture was incubated for 60 min at 37 °C, 95 % relative humidity, and 5% CO₂.

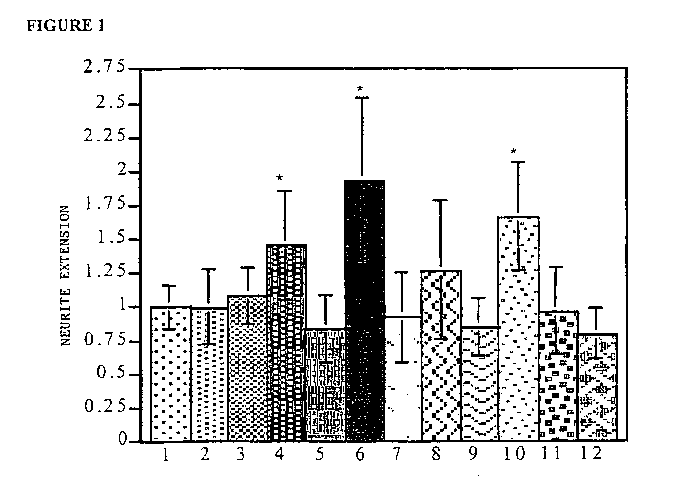

After polymerization, I ml TBS was added to each well. The gels were washed 5 times over 24 hr, with the first 4 wash solutions consisting of TBS and the last solution consisting of modified neural basal medium. Dorsal root ganglia (DRGs) were dissected from day 8 chick embryos and placed in Hanks-buffered salt solution. The ganglia were placed inside the fibrin gels (one per well) and the gels were incubated for 60 min at 37 °C, 95 % relative humidity, and 5% CO₂. After 60 min, I ml of modified neural basal medium was added to each well. The media was changed at 24 hr. Bright field images of the ganglia were taken at 24 and 48 hr. The images were analyzed to determine the average length of neurite extension, which was calculated to be the area of an annulus between the DRG body and the outer halo of extending neurites, as shown by Herbert et al. (1996) (23). Neurite extension for each experiment was normalized by the average neurite extension through unmodified fibrin gels from the same experiment and time point. Results are shown in Figure 1. Bar 1. This study demonstrates the utility of the invention as a cell support and growth material that will enhance neurite extension in three-dimensions. This is also demonstrated of the utility the invention would have for promoting cell growth and neurite extension under conditions found *in vivo.*

### EXAMPLE 3 -RELEASE OF BIOACTIVE NEUROTROPHINS FROM HEPARIN-BASED DELIVERY SYSTEM

An Example of a growth factor that is not considered to be heparin-binding growth factors, but that contains a basic sequence is nerve growth factor β (NGFβ) growth factor. This growth factor is considered to be non-heparin-binding and has even been used as a negative control for a heparin-binding protein in heparin-binding analysis (See Lee and Lander (1991) (24). However, NGF contains a basic domain at its C-terminus amino acid position (230-241 of human NGF beta) consisting of the following residues: CVLSRKAVRRA (SEQ ID NO:6). Similar basic domains can be found in the carboxy termini of neurotrophin-3 (NT-3, CALSRKIGRT, 248-257 of human NT-3, SEQ ID NO: 7) and brain-derived neurotrophic factor (CTLTIKRGR, 238-247 of human BDNF, SEQ ID NO: 8).

The present example demonstrates that NGFβ, NT-3 and BDNF can be delivered in a sustained manner from heparin-containing fibrin matrices that contain non-covalently immobilized heparin. This system consists of covalently immobilized heparin-binding peptides cross-linked to the fibrin matrix by factor XIIIa (See Schense and Hubbell (1999) (25) and heparin, which is non-covalently attached to these heparin-binding peptides. These materials have been shown by the present inventor to effectively deliver heparin-binding growth factors, such as basic fibroblast growth factor.

With this system, a bi-domain peptide is synthesized to comprise a heparin-binding sequence in one domain and a substrate for the coagulation enzyme factor XIIIa in the other domain (Schense and Hubbell, (1999) (25)). This enzyme covalently attaches the substrate domain of the bi-domain peptide to the fibrin network during coagulation, thereby also immobilizing the heparin-binding domain of the bi-domain peptide to the fibrin network. Heparin is also included into the coagulation mixture, and the heparin is thereby immobilized to the fibrin network by binding to the immobilized peptide.

The release system was first characterized with growth factors that demonstrate strong heparin binding affinity. The results of these studies are shown in Figure 1. Bar 1 shows the neurite extension through unmodified fibrin gels. Bar 2 shows that the release system, consisting of heparin and incorporated heparin-binding peptide, does not promote neurite extension without the addition of growth factor. Bars 3, 4 and 6 show the dose-response effect of matrix-bound bFGF, which enhances neurite extension by up to 100% (bar 6). Bars 5 and 7 show that when bFGF is added during polymerization of the fibrin gel in the absence of the release system, it does not enhance neurite extension, presumably because it diffuses out of the fibrin too quickly. Bar 8 shows that the presence of bFGF in the culture media promotes neurite extension similar to that of matrix bound bFGF at the same concentration. Bar 9 shows that VEGF does not enhance neurite extension, demonstrating that the growth factor bound must also show bioactivity in neural models to promote neurite extension. Bar 10 shows that when the amount of heparin-binding sites is decreased by 50%, the release of growth factor is not significantly affected. This deviation supports the contention that a high excess of growth factor binding sites is present. Bar 11 shows that when the peptide is cross-linked to the matrix, it will constitute a functional heparin-based delivery system. Bar 12 demonstrates that heparin and bFGF without the immobilized heparin-binding peptide do not constitute a functional delivery system and that heparin and immobilized peptide will enhance and sustain the sustained release of growth factor. These results demonstrate that the delivery system is capable of sustained release of a heparin binding growth factor in an active form.

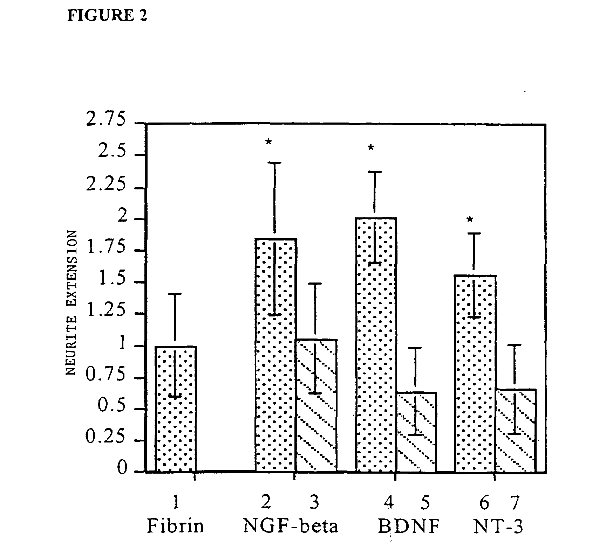

The ability of heparin-containing fibrin matrices to deliver low-heparin-binding growth factors has been tested using NGF-β, NT-3 and BDNF, all members of the neurotrophin family. Fibrin gels were made as described in Example 2, except for the addition of peptide, heparin and neurotrophin described below. Fibrin gels were made containing a final concentration of 3.5 mg/ml fibrinogen, 2.5 mM Ca++, 2 NIH units/ml thrombin. 0.25 mM peptide, 0.125 mM heparin, and 0.1 µg/ml of the neurotrophin to be tested. Otherwise, the assay was performed as described in Example 2. The results are shown in Figure 2. The fibrin group represents the behavior of normal fibrin and was the control data set to which neurite extension was normalized. These ganglia were cultured in the presence of 20 ng/ml NGF. All other treatments contained no growth factors in the media. For each of the three neurotrophins tested, neurite extension was enhanced only when the heparin-based delivery system of peptide and heparin was present and bound to the matrix ("bound" bars). The addition of neurotrophin alone ("soluble" bars) without peptide or heparin did not enhance neurite extension presumably because the factor diffused out of the fibrin too quickly. The materials shown in Figure 2 differ from those in Figure 1 in that "low-heparin-binding affinity" growth factors were released in Figure 2, while the factors released in Figure I are considered strongly heparin-binding. This distinction is clearly demonstrated in Example 1, in which the heparin-binding growth factor (bFGF) eluted from heparin above physiological NaCl concentrations, whereas the low and non-heparin-binding growth factors (NGF-β, and TGF-β2) eluted at sub-physiological NaCl concentrations. In both cases, the heparin-based delivery systems were able to deliver bioactive growth factors in a controlled manner. This demonstrates, surprisingly, that non-heparin-binding growth factors can be released in a sustained manner from heparin-containing matrices.

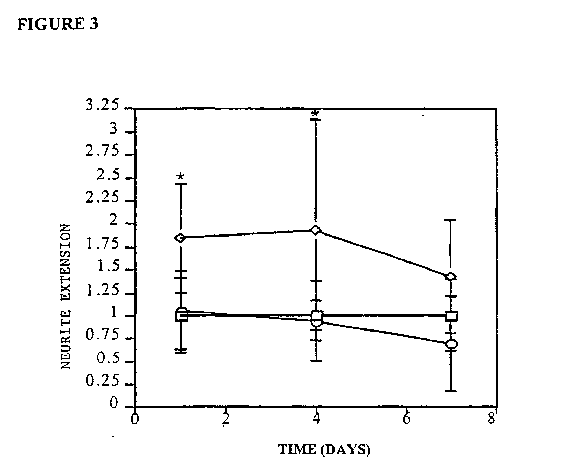

To determine how long the release of low-affinity growth factors could be sustained, NGF-β-containing gels were washed for extended time periods prior to cell seeding. In each case, the gels were washed thoroughly with TBS. The results are shown in Figure 3. The bioactivity of the growth factor present after 4 days is the same as after 1 day of washing. After 1 week of washing, the bioactivity of the growth factor released is decreased.

These results demonstrate that NGF-β, NT-3 and BDNF are being sequestered by heparin and that the increase in outgrowth versus unmodified fibrin is due to matrix bound growth factor, rather than free growth factor. This demonstrates that such heparin-based materials can be used to sequester proteins with low heparin-binding affinity, which contain exposed basic regions. In this three-dimensional fibrin matrix, heparin was immobilized at approximately 380 µg/ml. This material demonstrated NGF release over approximately one week. There are situations when release for as little as a day would be therapeutically useful. Thus a useful amount of immobilized heparin would be at least 95 µg/ml, with higher amounts leading to release sustained over longer periods.

The results shown here demonstrate that "non-heparin-binding growth factors", such as NGFβ, BDNF and NT-3, can be released in a controlled manner from heparin-based drug delivery systems based on their low affinity for heparin. These proteins can be sequestered within three-dimensional materials containing immobilized heparin based on basic domains found in the proteins. Furthermore, the growth factors released from these systems retain bioactivity in *in vitro* models as shown above. This demonstrates other low heparin-binding affinity growth factor proteins containing similar types of basic domains could be released from heparin-based delivery systems in a similar manner.

### EXAMPLE 4 - MEMBERS OF THE TGF-β FAMILY

The approach to sequence analysis described above can be applied to growth factors from other families as well. A list of growth factors and their sequences is shown in Table 2, which shows domains that may have low heparin-binding affinity and could be released from heparin-based delivery systems such as those described above. These factors include some members of the TGF-β family, namely TGF-β2, TGF-β3. and TGF-β4. TGF-β2. TGF-β3 and TGF-β4 are members of a family, which contains one strongly heparin-binding growth factor. TGF-β1. However, other members of the TGF-β family have been reported in the literature to have lower heparin-binding affinity, such as TGF-β3 (See Lyon et al (1997) (26). TGF-β1 has been shown to be heparin-binding at physiological ionic strength, i.e. at 140 mM NaC1. TGF-β3 lacks a key charge and has been demonstrated to be "non-heparin-binding" at physiological conditions. Heparin-affinity chromatography on TGF-β2 by the present inventors demonstrates that it also possesses low-heparin-binding affinity under physiological conditions. The basic domain in each of these growth factors, which could potentially interact with heparin, is underlined in the list of sequences given in Table 2.

Some members of a growth factor family may be heparin-binding, while others are not, as is demonstrated by the TGF-β family. Despite the low-heparin-binding affinity of such "non-heparin-binding" growth factors, they may still be released in a controlled manner from heparin-based delivery systems if they contain a basic domain and the number of heparin-binding sites present in the system is in relatively greater excess to the amount of growth factor to be bound.

### EXAMPLE 5 - OTHER "NON-HEPARIN-BINDING" GROWTH FACTORS WHICH CONTAIN BASIC DOMAINS

Other families of growth factors also contain growth factors which are not reported in the literature to be heparin-binding, but which contain basic domains(Shown in Table 2.

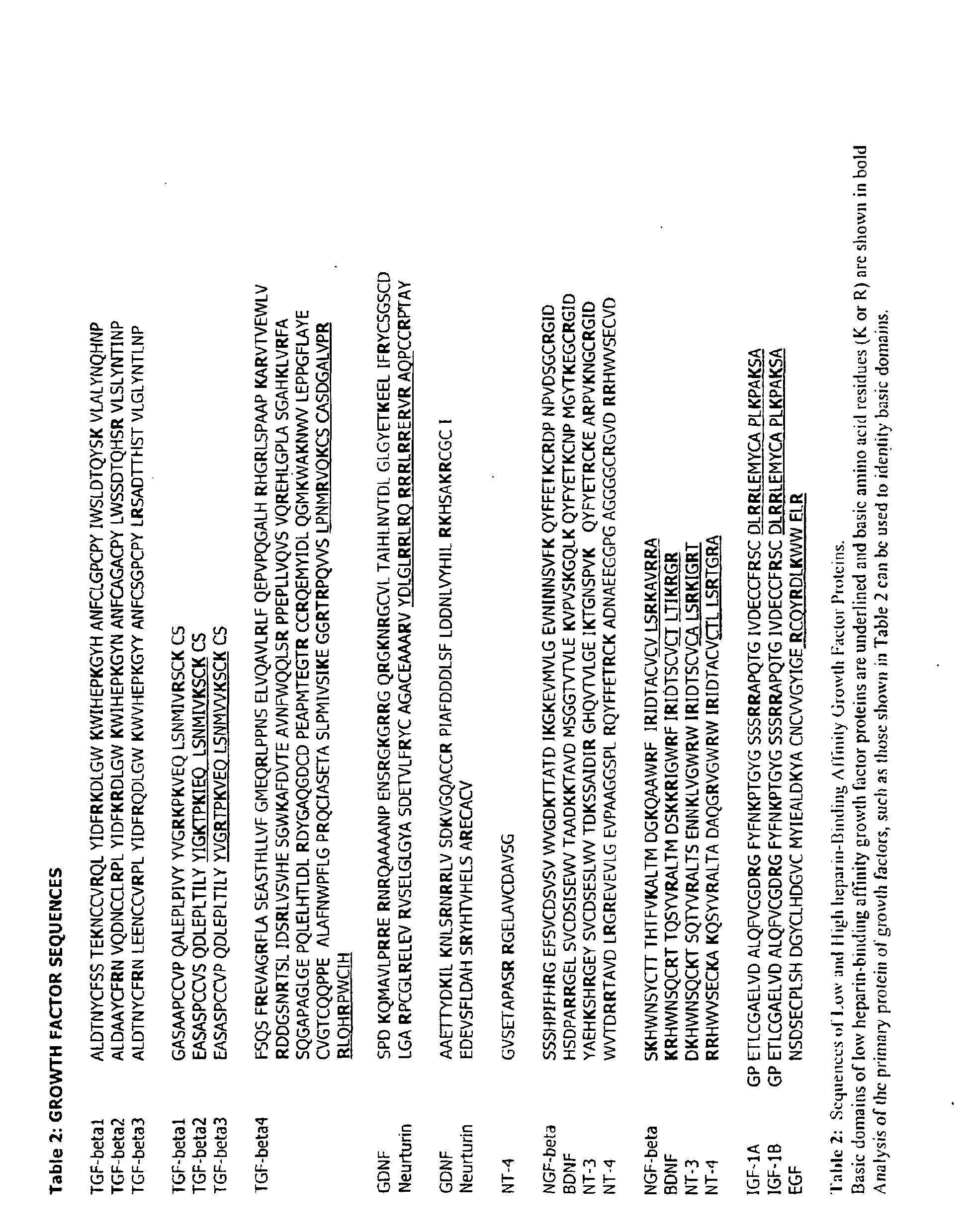

By analyzing the sequences of the TGFβ family and the neurotrophin family, one skilled in the art can observe a pattern in the basic domains underlined in Table 2. From this pattern observed in the basic domains of low heparin-binding affinity proteins, an approximate formula was developed to identify similar basic domains in other proteins. The formula defines a basic domain to be of length about 8 to 30 amino acid residues, comprising at least 2 basic amino acid residues, with a ratio of basic to acidic amino acid residues to at least 2, and a ratio of hydrophobic amino acid residues of at least 0.67. Secondary and tertiary protein structure also influences the heparin-binding affinity of a basic domain within a protein or peptide, and for this reason the formula is approximate. Therefore, it is necessary to perform heparin-affinity chromatography or some other experimental technique to determine the relative heparin affinity of a protein or peptide. The term "low heparin-binding affinity protein" refers only to those proteins or peptides which elute from heparin-affinity chromatography at a NaCI concentration of less than about 140 mM. The formula for sequence analysis described above was applied to the list of growth factors found in Table 2 and used to identify basic domains which could potentially bind to heparin or heparin-like polymers.

Low heparin-binding affinity protein should dissociate rapidly from heparin under physiological conditions. However, surprisingly the results shown in Fig. 3 demonstrate that such low heparin-binding affinity growth factor proteins can be released in a controlled manner from heparin-based delivery systems. This novel result suggests that other growth factors containing domains which meet the requirements of the formula described above may also be released in a controlled manner from heparin-based delivery systems. Examples of such growth factors are shown in the sequence list in Table 2, and the basic domains of these growth factors are underlined.

Two members of the GDNF family, namely neurturin and persephin, both contain basic domains. GDNF is reported in the literature to be heparin-binding, but no reports have been made to date on the heparin-affinity of other members of the GDNF family (27). Neurturin and persephin, based on the analysis presented above, would appear to have sufficient heparin binding affinity to be releasable by the methods and materials of this invention.

IGF-1A and IGF-1B are members of the insulin-like growth factor family. Although there are extensive reports of insulin-like growth factor binding proteins binding to heparin, there is no documentation in the literature of IGF-1A or IGF-1B binding to heparin. Both of these proteins contain basic domains shown in Table 2. Based on the analysis presented above, would appear to have sufficient heparin binding affinity to be releasable by the methods and materials of this invention.

EGF is another growth factor, which contains a basic domain, shown in Table 2. but which is not reported in the literature to be heparin-binding. In fact, the existence of a related growth factor specifically referred to as heparin-binding EGF-like growth factor suggests that EGF does not possess high heparin-affinity. The literature suggests EGF is not heparin-binding based on sequence analysis (45). Based on the analysis presented above, it appears to the present inventors to have sufficient heparin binding affinity to be releasable by the methods and materials of this invention.

Despite the basic domains found in each of these proteins, their heparin-binding affinity is still weak and characterized by elution from heparin-affinity columns at sub-physiological NaCl concentrations (i.e. growth factors which elute between about 25 mM and 140 mM NaCl). For example, heparin-affinity chromatography was performed for NGF-β and TGF-β2. In both cases the proteins were found to elute at 50 mM NaCl at pH 7.4 after several column volumes of buffer, which is well below the physiological NaC 1 concentration. However, *in vitro* studies demonstrate that heparin-based release systems can still be used to release NGF-β in a controlled manner, in spite of its relatively low heparin-binding affinity.

All of the growth factors described in the example contain basic domains, but lack any literature reports of heparin-binding affinity. However, based on comparison of the sequences and the results demonstrated with other non-heparin-binding growth factors of the neurotrophin family, sustained release from heparin-based systems should be possible with virtually any growth factor having a basic domain having the characteristics of basic domains described above.

### BIBLIOGRAPHY

1. Presta, M., Satuto. M., Isacchi, A., Caccia, P., Pozzi. A., Gualandris, A., Rusnati, M., Bergonzoni. L., and Sarmientos, P. (1992) *Biochem Biophys Res Commun* 185: 1098-1107
2. Reddi, A. (1998) *Nature Biotechnology* 16: 247-252
3. McCaffrey, T., Falcone. D., and Du, B. (1992) *J Cell Physiol* 152: 430-440
4. Spillmann, D., Witt, D., and Lindahl, U. (1998) *J Biol Chem* 273: 15487-15493
5. Götz, R., Köster, R., Winkler, C., Raulf. F., Lottspeich, F., Schartl, M., and Thoenen, H. (1994) *Nature* 372: 266-269
6. Tessler, S., Rockwell, P., Hicklin, D., Cohen, T., Levi, B., Witte, L., Lemischka, I., and Neufeld, G. (1994). *J Biol Chem* 269: 12456-12461
7. Kiguchi, K., Beltran, L., Rupp, T., and DiGiovanni, J. (1998) *Mol Carcinog* 22: 73-83
8. Kinosaki, M., Yamagucki, K., Murakami. A., Ueda, M., Morinaga, T., and Higashio, K. (1998) *Biochim Biophys Acta* 1384: 93-102
9. Steffen, C., Ball-Mirth. D.. Harding, P., Bhattacharyya, N., Pillai, S., and Brigstock. D. (1998) *Growth Factors* 15: 199-213
10. Kaneda, N., Talukder, A., Nishiyama, H., Koizumi, S., and Muramatsu, T. (1996) *J Biochem (Tokyo)* 119: 1150-1156
11. Nolo, R., Kaksonen, M., and Rauvala, H. (1996). *Eur J Neurosci* 8: 1658-1665
12. Edelman, E.. Mathiowitz, E.. Langer, R., and Klagsbrun, M. (1991) *Biomaterials* 12: 612-626
13. DeBlois, C., Cote, M.-F., and Doillon, C. (1994) B*iomaterials* 15: 665-672
14. Downs, E., Robertson, N., Riss, T., and Plunkett, M. (1992) *J Cell Physiol* 152: 422-429
15. Houle, J. and Johnson, J. (1989) *Neurosci Lett* 103: 17-23
16. Camarata, P., Suryanarayanan, R., Turner, D., Parker, R., and Ebner, T. (1992) *Neurosurgery* 30: 313-319
17. Powell, E., Sobarzo, M., and Saltzman. W. (1990) *Brain Res* 515: 309-311
18. Maysinger, D., Jalenjak, I., and Cuello, A. (1992) *Neurosci Lett* 140: 71-74
19. Edelman, E., Langer, R., Klagsbum, M., and Mathiowitz, E. (1992) *Controlled Release Systems Containing Heparin and Growth Factors,* MIT: USA
20. Schroeder, J., Bentz. H., and Estridge, T. (1997) *Affinity bound collagen matrices for the delivery of biologically active agents,* Collagen Corporation: USA.
21. Schroeder-Tefft, J., Bentz, H., and Estridge, T. (1997) *J Controlled Release* 49: 291-298
22. Cardin, A. and Weintraub, H. (1989) *Atherosclerosis* 9: 21-32
23. Herbert. C., Bittner, G., and Hubbell. J. (1996) *J Comp Neurol* 365: 380-391
24. Lee, M. and Lander. A. (1991) *Proc Natl Acad Sci U S A* 88: 2768-2772
25. Schense, J. and Hubbell, J. (1999) *Bioconjug Chem* 10: 75-81
26. Lyon, M., Rushton, G., and Gallagher, J. (1997) *J Biol Chem* 272: 1800-18006
27. Lin, L.-F.H., Zhang, T., Collins, F., and Armes, L. (1994) *J Neurochem* 63: 758-768
28. Besson, C. Saulnier J., Wallach, J. *(1996) Analytical Biochemistry* 237: 216-223
29. Coombs G., Bergstrom R., Madison. E.. and Corey, D. (1998) *The Journal of Biological Chemisty* 237: 4323-4328
30. G ötz, R., Köster, R., Winkler, C., Raulf, F., Lottspeich, F., Shartl, M., and Thoenen, H. (1994) *Nature* 372: 266-269
31. Hata, A., Ridinger, D.. Sutherland, S., Emi, M., Shuhua, Z., Myers, R., Ren, K., Cheng, T., Inoue, I., Wilson, D., Iverius, P., and Lalouel. J. (1993) *The Journal of Biological Chemistry* 268: 8847-8457
32. Haugen, P., McCarthy, J., Roche, K., Furcht, L., and Letourneau, P. (1992) *The Journal of Neuroscience* June 2034-2042
33. Kallapur, S. and Akeson, R. (1992) *Journal of Neuroscience Research* 33: 538-548
34. Kaneda, N., Talukder, A., Nishiyama, H., Koizumi, S., and Muramatsu, T. (1996) *J. Biochem* 119: 1150-1156
35. Kiguchi, K., Beltrán, L.. Rupp, T., and DiGiovanni, J. (1998) *Molecular Carcinogenesis* 22: 73-83
36. Kinosaki, M., Yamaguchi, K., Murakami, A., Ueda, M., Morinaga, T., Higashio, K. (1998) *Biochimica et Biophysica Acta* 1384: 93-102
37. McCaffrey, T., Falcone, D., and Du, B. (1992) *Journal of Cellular Physiology* 152: 430-440
38. Netzel-Amett, S.. Fields. G., Birkedal-Hansen, H., and Van Wart. H. (1991) *The Journal of Biological Chemistry* 266: 6747-6755
39. Nolo, R., Kaksonen. M. and Rauvala. H. (1996) *European Journal of Neuroscience* 8: 1658-1665
40. Smith, M., Shi,L. and Navre, M. (1995) *The Journal of Biological Chemistry* 270: 6440-6449
41. Spillmann, D., Witt, D., and Lindahl, U. (1998) *The Journal of Biological Chemistry* 273: 15487-15493
42. Steffen, C., Ball-Mirth, D., Harding, P., Bhattacharyya, N., Pillai, S.. and Brigstock, D. (1998) *Growth Factors* 15: 199-213
43. Presta, et al. (1992) *Biochemical and Biophysical Research Communications,* 185: 1098-1107 (1992)
44. Zucker, M. and Katz, I., (1991) *Society for Experimental Biology and Medicine:* 693-702
45. Higashiyama S., Abraham J. A., Miller J.. Fiddes, J. C., and Kiagsbrun, M. (1991) *Science 251 (4996):* 936-9
46. Maaroufi, R. M.. Jozefowicz, M., Tapon-Bretaudiere J., Jozefonwicz, J., Fischer, A. M., Biomaterials 1997, 18:359-366.
47. Logeart. D., Prrgent-Richard, S., Jozefonwicz, J., Letourneur, D., Eur J Cell Biol 1997, 74:376-384.
48. de Raucourt, E., Maural, S., Chaubet. F., Maiga-Revel, O., Jozefonwicz, M., Fischer, A. M., J. Biomed Mater Res 1998, 41 :49-57
49. Bagheri-Yamand, R., Kourbali, Y., Mabilat, C., Morere, J. F., Martin, A., Lu, H., Soria, C., Jozefonwicz, J., Crepin. M., Br. J. Cancer 1998, 78:111-118.
50. Silver, J. H., Hart, A. P., Williams, E. C.. Cooper S. L.. Charef, S., Labarre, D., Jozefowicz, M., Biomaterials 1992, 13:339-344.
51. U.S. Patent No. 5,830,700 Irani. Meher

## Claims

1. A delivery system for a protein growth factor or peptide fragment thereof, comprising
- a substrate,
- a peptide which is covalently bound to the substrate, and which has a binding domain that binds heparin or a heparin-like polysaccharide or a heparin-like polymer with high affinity, wherein high affinity is defined as eluting from a heparin-affinity column at not less than 140 mM NaCl,
- heparin or a heparin-like polysaccharide or a heparin-like polymer bound to that peptide,
- a protein growth factor or peptide fragment thereof having a domain that binds said heparin or heparin-like polysaccharide or heparin-like polymer with low affinity, wherein low affinity is defined as providing elusion from a heparin-affinity column at a NaCl concentration of less than about 140 mM.

2. The delivery system of claim 1 wherein the protein growth factor or peptide fragment thereof is defined as eluting from a heparin-affinity column at a NaCl concentration of about 25 mM to about 140 mM.

3. The delivery system of claim 1 or 2 wherein the domain of the protein growth factor or peptide fragment thereof is further defined as comprising a length of about 8 to 30 amino acid residues comprising at least 2 basic amino acid residues, a ratio of basic to acidic amino acid residues of at least 2, and a ratio of hydrophobic amino acid residues to basic amino residues of at least 0.67.

4. The delivery system of claim 3 wherein the basic amino acid residues are K or R.

5. The delivery system of claim 3 or 4 wherein the acidic amino acid residues are further defined as D or E.

6. The delivery system of anyone of claims 3 to 5 wherein the hydrophobic amino acid residues are further defined as A,V,F,P,M,I, or L or C when C is involved in a disulfide bond.

7. The delivery system of anyone of claims 1 to 6 wherein the protein growth factor or peptide fragment thereof is neurturin, persephin, IGF-1A, IGF-1β, EGF,NGFβ, NT-3, BDNF, NT-4, TGF-β2, TGF-β3, or TGF-β4.

8. The delivery system of anyone of claims 1 to 7 wherein the substrate comprises at least one of
fibrin, collagen, hyaluronic acid, hyaluronic acid derivatives, and synthetic polymer hydrogels.

9. The delivery system of claim 8 wherein the substrate is fibrin.

10. The delivery system of anyone of claims 1 to 9 wherein the heparin or heparin-like polysaccharide or heparin-like polymer is non-covalently attached to the peptide that binds heparin or a heparin-like polysaccharide or heparin-like polymer with high affinity.

11. The delivery system of anyone of claims 1 to 10 wherein the peptide that binds heparin or a heparin-like polysaccharide or a heparin-like polymer with high affinity is further defined as comprising SEQ ID NO:1, SEQ ID NO:2, SEQ ID NO:3, SEQ ID NO:4, or SEQ ID NO:5.

12. The delivery system of anyone of claims 1 to 11 wherein the heparin or heparin-like polysaccharide or heparin-like polymer has a molecular weight between about 3000 and 10 000 000 Daltons.

13. The delivery system of anyone of claims 1 to 12 wherein the heparin-like polysaccharide has a molecular weight between about 3000 and 10 000 000 Daltons, and has at least one negative charge per 2 saccharide rings and no more than one positive charge per 10 saccharide rings.

14. The delivery system of anyone of claims 1 to 13 wherein the heparin-like polymer is dextran sulfate, chondroitin sulfate, heparan sulfate, fucan, alginate or a derivative thereof.

15. The delivery system of anyone of claims 1 to 14 wherein the molar ratio of heparin or heparin-like polysaccharide or heparin-like polymer to protein growth factor or peptide fragment thereof is at least 1.

16. The delivery system of anyone of claims 1 to 15 wherein the molar ratio of covalently attached peptide that binds heparin or a heparin-like polysaccharide or a heparin-like polymer with high affinity to heparin or heparin-like polysaccharide or heparin-like polymer is at least 1.

17. An article of manufacture comprising a delivery system for a protein growth factor or a peptide fragment thereof according to anyone of claims 1 to 16 wherein the substrate is capable of supporting cell adhesion.

18. The article of manufacture of claim 17 which is a vascular graft.

19. The article of manufacture of claim 17 which is an article for treatment of dermal wounds.

20. The article of manufacture of claim 17 which is an implantable sterilized composition.

21. Use of a peptide which is able to covalently bind to a substrate and which has a binding domain that binds heparin or a heparin-like polysaccharide or a heparin-like polymer with high affinity, wherein high affinity is defined as eluting from a heparin-affinity column at not less than 140 mM NaCl, for the manufacture of a delivery system according to any one of claims 1 to 16.

22. The use of claim 21 wherein the protein growth factor or peptide factor comprised in the delivery system is released by dissociation of the protein growth factor or peptide factor thereof from the heparin or heparin-like polysaccharide or heparin-like polymer.

## Patentansprüche

1. Zuführsystem für einen Protein-Wachstumsfaktor oder ein Peptid-Fragment davon, aufweisend
- ein Substrat,
- ein Peptid, das kovalent an das Substrat gebunden ist, und das eine Bindungsdomäne hat, die Heparin oder ein Heparin-ähnliches Polysaccharid oder ein Heparin-ähnliches Polymer mit hoher Affinität bindet, wobei hohe Affinität definiert ist als bei nicht weniger als 140 mM NaCl aus einer Heparin-Affinitätssäule eluierend,
- Heparin oder ein Heparin-ähnliches Polysaccharid oder ein Heparin-ähnliches Polymer, das an das Peptid gebunden ist,
- einen Protein-Wachstumsfaktor oder ein Peptid-Fragment davon mit einer Domäne, die das Heparin oder das Heparin-ähnliche Polysaccharid oder das Heparin-ähnliche Polymer mit geringer Affinität bindet, wobei geringe Affinität definiert ist als für Eluieren aus einer Heparin-Affinitätssäule bei einer NaCl-Konzentration von weniger als etwa 140 mM sorgend.

2. Zuführsystem nach Anspruch 1, bei dem der Protein-Wachstumsfaktor oder das Peptid-Fragment davon definiert ist als aus einer Heparin-Affinitätssäule bei einer NaCl-Konzentration von etwa 25 mM bis etwa 140 mM eluierend.

3. Zuführsystem nach Anspruch 1 oder 2, bei dem die Domäne des Protein-Wachstumsfaktors oder des Peptid-Fragments davon außerdem definiert ist als eine Länge von etwa 8 bis 30 Aminosäureresten aufweisend, die mindestens zwei basische Aminosäurereste, ein Verhältnis von basischen zu sauren Aminosäureresten von mindestens 2, und ein Verhältnis von hydrophoben Aminosäureresten zu basischen Aminosäureresten von mindestens 0,67 aufweisen.

4. Zuführsystem nach Anspruch 3, bei dem die basischen Aminosäurereste K oder R sind.

5. Zuführsystem nach Anspruch 3 oder 4, bei dem die sauren Aminosäurereste außerdem als D oder E definiert sind.

6. Zuführsystem nach einem der Ansprüche 3 bis 5, bei dem die hydrophoben Aminosäurereste außerdem definiert sind als A, V, F, P, M, I, oder als L oder C, wenn C an einer Disulfid-Bindung beteiligt ist.

7. Zuführsystem nach einem der Ansprüche 1 bis 6, bei dem der Protein-Wachstumsfaktor oder das Peptid-Fragment davon Neurturin, Persephin, IGF-1A, IGF-1β, EGF, NGFβ, NT-3, BDNF, NT-4, TGF-β2, TGF-β3 oder TGF-β4 ist.

8. Zuführsystem nach einem der Ansprüche 1 bis 7, bei dem das Substrat mindestens eine der Komponenten Fibrin, Kollagen, Hyaluronsäure, Hyaluronsäure-Derivate und synthetische Polymer-Hydrogele aufweist.

9. Zuführsystem nach Anspruch 8, bei dem das Substrat Fibrin ist.

10. Zuführsystem nach einem der Ansprüche 1 bis 9, bei dem das Heparin oder das Heparin-ähnliche Polysaccharid oder das Heparin-ähnliche Polymer nicht-kovalent an dem Peptid, das Heparin oder ein Heparin-ähnliches Polysaccharid oder ein Heparin-ähnliches Polymer mit hoher Affinität bindet, angebracht ist.

11. Zuführsystem nach einem der Ansprüche 1 bis 10, bei dem das Peptid, das Heparin oder ein Heparin-ähnliches Polysaccharid oder ein Heparin-ähnliches Polymer mit hoher Affinität bindet, außerdem definiert ist als SEQ ID NO:1, SEQ ID NO:2, SEQ ID NO:3, SEQ ID NO:4 oder SEQ ID NO:5 aufweisend.

12. Zuführsystem nach einem der Ansprüche 1 bis 11, bei dem das Heparin oder das Heparin-ähnliche Polysaccharid oder das Heparin-ähnliche Polymer ein Molekulargewicht zwischen etwa 3.000 und 10.000.000 Dalton hat.

13. Zuführsystem nach einem der Ansprüche 1 bis 12, bei dem das Heparin-ähnliche Polysaccharid ein Molekulargewicht zwischen etwa 3.000 und 10.000.000 Dalton hat und mindestens eine negative Ladung pro 2 Saccharidringen und nicht mehr als eine positive Ladung pro 10 Saccharidringen hat.

14. Zuführsystem nach einem der Ansprüche 1 bis 13, bei dem das Heparin-ähnliche Polymer Dextransulfat, Chondroitinsulfat, Heparansulfat, Fucan, Alginat oder ein Derivat davon ist.

15. Zuführsystem nach einem der Ansprüche 1 bis 14, bei dem das Molverhältnis von Heparin oder Heparin-ähnlichem Polysaccharid oder Heparin-ähnlichem Polymer zu Protein-Wachstumsfaktor oder Peptid-Fragment davon mindestens 1 beträgt.

16. Zuführsystem nach einem der Ansprüche 1 bis 15, bei dem das Molverhältnis von kovalent angebrachtem Peptid, das Heparin oder ein Heparin-ähnliches Polysaccharid oder ein Heparin-ähnliches Polymer mit hoher Affinität bindet, zu Heparin oder Heparin-ähnlichem Polysaccharid oder Heparin-ähnlichem Polymer mindestens 1 beträgt.

17. Fertigungsgegenstand, aufweisend ein Zuführsystem für einen Protein-Wachstumsfaktor oder ein Peptid-Fragment davon nach einem der Ansprüche 1 bis 16, bei dem das Substrat in der Lage ist, eine Zellanhaftung zu unterstützen.

18. Fertigungsgegenstand nach Anspruch 17, der ein Gefäßtransplantat ist.

19. Fertigungsgegenstand nach Anspruch 17, der ein Gegenstand zur Behandlung von Hautwunden ist.

20. Fertigungsgegenstand nach Anspruch 17, der eine implantierbare sterilisierte Zusammensetzung ist.

21. Verwendung eines Peptids, das in der Lage ist, kovalent an ein Substrat zu binden, und das eine Bindungsdomäne hat, die Heparin oder ein Heparin-ähnliches Polysaccharid oder ein Heparin-ähnliches Polymer mit hoher Affinität bindet, wobei hohe Affinität definiert ist als aus einer Heparin-Affinitätssäule bei nicht weniger als 140 mM NaCl eluierend, zur Herstellung eines Zuführsystems nach einem der Ansprüche 1 bis 16.

22. Verwendung nach Anspruch 21, wobei der in dem Zuführsystem enthaltene Protein-Wachstumsfaktor oder Peptid-Faktor durch Abspaltung des Protein-Wachstumsfaktors oder Peptid-Faktors davon von dem Heparin oder Heparin-ähnlichem Polysaccharid oder Heparin-ähnlichem Polymer freigesetzt wird.

## Revendications

1. Système de délivrance d'un facteur de croissance protéinique ou fragment peptidique de celui-ci, comprenant
- un substrat,
- un peptide qui est lié par covalence au substrat, et qui a un domaine de liaison qui se lie à l'héparine ou à un polysaccharide héparinoïde ou à un polymère héparinoïde avec une forte affinité, la forte affinité étant définie par une élution à partir d'une colonne d'affinité de type héparine à pas moins de 140 mM de NaCl,
- une héparine ou un polysaccharide héparinoïde ou un polymère héparinoïde lié à ce peptide,
- un facteur de croissance protéinique ou fragment peptidique de celui-ci ayant un domaine qui se lie à ladite héparine ou audit polysaccharide héparinoïde ou polymère héparinoide avec une basse affinité, la basse affinité étant définie par une élution à partir d'une colonne d'affinité de type héparine à une concentration de NaCl inférieure à environ 140 mM.

2. Système de délivrance selon la revendication 1, dans lequel le facteur de croissance protéinique ou fragment peptidique de celui-ci est défini par une élution à partir d'une colonne d'affinité de type héparine à une concentration de NaCl d'environ 25 mM à environ 140 mM.

3. Système de délivrance selon la revendication 1 ou 2, dans lequel le domaine du facteur de croissance protéinique ou fragment peptidique de celui-ci est défini, en outre, par une longueur d'environ 8 à 30 résidus acides aminés comprenant au moins 2 résidus acides aminés basiques, un rapport des résidus acides aminés basiques à acides d'au moins 2, et un rapport résidus acides aminés hydrophobes à résidus acides aminés basiques d'au moins 0,67.

4. Système de délivrance selon la revendication 3, dans lequel les résidus acides aminés basiques sont K ou R.

5. Système de délivrance selon la revendication 3 ou 4, dans lequel les résidus acides aminés acides sont, en outre, définis comme D ou E.

6. Système de délivrance selon l'une quelconque des revendications 3 à 5, dans lequel les résidus acides aminés hydrophobes sont, en outre, définis comme A, V, F, P, M, I, ou L ou C quand C est impliqué dans une liaison disulfure.

7. Système de délivrance selon l'une quelconque des revendications 1 à 6, dans lequel le facteur de croissance protéique ou fragment peptidique de celui-ci est la neurturine, la perséphine, IGF-1A, IGF-1β, EGF, NGFβ, NT-3, BDNF, NT-4, TGF-β2, TGF-β3, ou TGF-β4.

8. Système de délivrance selon l'une quelconque des revendications 1 à 7, dans lequel le substrat comprend au moins une substance parmi fibrine, collagène, acide hyaluronique, dérivés de l'acide hyaluronique, et hydrogels de type polymère synthétique.

9. Système de délivrance selon la revendication 8, dans lequel le substrat est la fibrine.

10. Système de délivrance selon l'une quelconque des revendications 1 à 9, dans lequel l'héparine ou le polysaccharide héparinoïde ou le polymère héparinoïde est lié de manière non covalente au peptide qui se lie à l'héparine, au polysaccharide héparinoïde ou au polymère héparinoïde avec une forte affinité.

11. Système de délivrance selon l'une quelconque des revendications 1 à 10, dans lequel le peptide qui se lie à l'héparine, au polysaccharide héparinoïde ou au polymère héparinoïde avec une forte affinité est, en outre, défini comme comprenant les SEQ ID N°1, SEQ ID N°2, SEQ ID N°3, SEQ ID N°4, ou SEQ ID N°5.

12. Système de délivrance selon l'une quelconque des revendications 1 à 11, dans lequel l'héparine, le polysaccharide héparinoïde ou le polymère héparinoïde a une masse moléculaire entre environ 3000 et 10 000 000 Daltons.

13. Système de délivrance selon l'une quelconque des revendications 1 à 12, dans lequel le polysaccharide héparinoïde a une masse moléculaire entre environ 3000 et 10 000 000 Daltons, et porte au moins une charge négative pour 2 cycles saccharide et pas plus d'une charge positive pour 10 cycles saccharide.

14. Système de délivrance selon l'une quelconque des revendications 1 à 13, dans lequel le polymère héparinoïde est le dextrane-sulfate, le chondroïtine-sulfate, l'héparane-sulfate, le fucane, l'alginate ou un dérivé de ceux-ci.

15. Système de délivrance selon l'une quelconque des revendications 1 à 14, dans lequel le rapport molaire de l'héparine ou du polysaccharide héparinoïde ou du polymère héparinoïde au facteur de croissance protéinique ou fragment de celui-ci est d'au moins 1.

16. Système de délivrance selon l'une quelconque des revendications 1 à 15, dans lequel le rapport molaire du peptide lié par covalence, qui se lie à l'héparine ou à un polysaccharide héparinoïde ou à un polymère héparinoïde, à l'héparine, au polysaccharide héparinoïde ou au polymère héparinoide est d'au moins 1.

17. Article fabriqué comprenant un système de délivrance d'un facteur de croissance protéinique ou fragment peptidique de celui-ci selon l'une quelconque des revendications 1 à 16, dans lequel le substrat est capable de supporter l'adhérence cellulaire.

18. Article fabriqué selon la revendication 17 qui est un greffon vasculaire.

19. Article fabriqué selon la revendication 17 qui est un article pour le traitement des lésions cutanées.

20. Article fabriqué selon la revendication 17 qui est une composition stérile implantable.

21. Utilisation d'un peptide qui est capable de se lier par covalence à un substrat et qui a un domaine de liaison qui se lie à l'héparine ou à un polysaccharide héparinoïde ou à un polymère héparinoïde avec une forte affinité, la forte affinité étant définie par une élution à partir d'une colonne d'affinité de type héparine à pas moins de 140 mM de NaCl, pour la fabrication d'un système de délivrance selon l'une quelconque des revendications 1 à 16.

22. Utilisation selon la revendication 21, dans laquelle le facteur de croissance protéinique ou le facteur peptidique contenu dans le système de délivrance est délivré par dissociation du facteur de croissance protéique ou facteur peptidique de celui-ci d'avec l'héparine, le polysaccharide héparinoïde ou le polymère héparinoïde.
